(19) Europäisches Patentamt
European Patent Office
Office européen des brevets

(11) EP 1 560 806 B1

(12) **EUROPÄISCHE PATENTSCHRIFT**

(45) Veröffentlichungstag und Bekanntmachung des Hinweises auf die Patenterteilung:
**10.01.2007 Patentblatt 2007/02**

(21) Anmeldenummer: **03779847.7**

(22) Anmeldetag: **05.11.2003**

(51) Int Cl.:
*C07C 211/40* (2006.01) *C07D 209/14* (2006.01)
*A61K 31/135* (2006.01) *A61P 25/00* (2006.01)

(86) Internationale Anmeldenummer:
**PCT/EP2003/012314**

(87) Internationale Veröffentlichungsnummer:
**WO 2004/043900 (27.05.2004 Gazette 2004/22)**

(54) **4-ALKYL-/4-ALKENYL-/4-ALKINYLMETHYL/-1-ARYLCYCLOHEXYLAMIN-DERIVATE**

4-ALKYL/4-ALKENYL/4-ALKYNYL METHYL/-1-ARYLCYCLOHEXYLAMINE DERIVATIVES

DERIVES DE 4-ALKYL-/4-ALCENYL-/4-ALCYNYLMETHYL-/1-ARYL-CYCLOHEXYLAMINE

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **12.11.2002 DE 10252874**
**14.11.2002 DE 10253322**

(43) Veröffentlichungstag der Anmeldung:
**10.08.2005 Patentblatt 2005/32**

(73) Patentinhaber: **Grünenthal GmbH**
**52078 Aachen (DE)**

(72) Erfinder:
- **SUNDERMANN, Bernd**
  **52066 Aachen (DE)**
- **SCHICK, Hans**
  **10405 Berlin (DE)**
- **HINZE, Claudia**
  **52066 Aachen (DE)**

(56) Entgegenhaltungen:
- **KAWAMOTO H ET AL: "Synthesis of J-113397, the first potent and selective ORL1 antagonist" TETRAHEDRON, ELSEVIER SCIENCE PUBLISHERS, AMSTERDAM, NL, Bd. 57, Nr. 6, 4. Februar 2001 (2001-02-04), Seiten 981-986, XP004316527 ISSN: 0040-4020**
- **MEUNIER J-C ET AL: "ISOLATION AND STRUCTURE OF THE ENDOGENOUS AGONIST OF OPIOID RECEPTOR-LIKE ORL1 RECEPTOR" NATURE, MACMILLAN JOURNALS LTD. LONDON, GB, Bd. 377, 12. Oktober 1995 (1995-10-12), Seiten 532-535, XP002020240 ISSN: 0028-0836 in der Anmeldung erwähnt**
- **VONVOIGTLANDER F ET AL: "4-(p-Bromophenyl)-4-(dimethylamino)-1-phenethylcyclohexanol, an Extremely Potent Representative of a New Analgesic Series" JOURNAL OF MEDICINAL CHEMISTRY, AMERICAN CHEMICAL SOCIETY. WASHINGTON, US, Bd. 22, Nr. 10, 1979, Seiten 1157-1158, XP002216903 ISSN: 0022-2623**

EP 1 560 806 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft 4-Alkyl-/4-Alkenyl-/4-Alkinylmethyl/-1-aryl-cyclohexylamin-Derivate, Verfahren zu deren Herstellung, Arzneimittel enthaltend diese Verbindungen und die Verwendung von 4-Alkyl-/4-Alkenyl-/4-Alkinylmethyl/-1-aryl-cyclohexylamin-Derivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist.
Der ORL1-Rezeptor ist homolog zu den μ, κ und δ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581),
Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen gerade im Bereich der Schmerztherapie, aber auch bei anderen der genannten Indikationen, Opioidrezeptoren wie der μ-Rezeptor und andere Subtypen eine große Rolle. Entsprechend ist es günstig, wenn die Verbindung auch Wirkung an diesen Opioidrezeptoren zeigen.

Aus der Literatur ist bereits die Verbindung 4-(p-Bromphenyl)-4-(dimethylamino)-1-phenethylcyclohexanol als potentes Analgetikum bekannt (J. Med. Chem 1979, 22, 1157-1158). Die erfindungsgemäßen Verbindungen unterscheiden sich dadurch, dass sie in 4-Position des Cyclohexanringes keine OH-Gruppe tragen.

Weiterhin ist aus der Literatur ein trisubstituiertes Piperidinderivat bekannt, das an den ORL-1-Rezeptor bindet (Tetrahedron 2001, 57, 981-986). Die erfindungsgemäßen Verbindungen unterscheiden sich dadurch, dass sie einen Cyclohexanring anstelle eines Piperidinrings enthalten, der in 3-Position keine Hydroxygruppe trägt. Die Substituenten in 1- und 4-Positon des Sechsrings sind ebenfalls unterschiedlich definiert.

Aufgabe der vorliegenden Erfindung war es, Arzneimittel zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Gegenstand der Erfindung sind daher 4-Alkyl-/4-Alkenyl-/4-Alkinylmethyl/-1-aryl-cyclohexylamin-Derivate der allgemeinen Formel I,

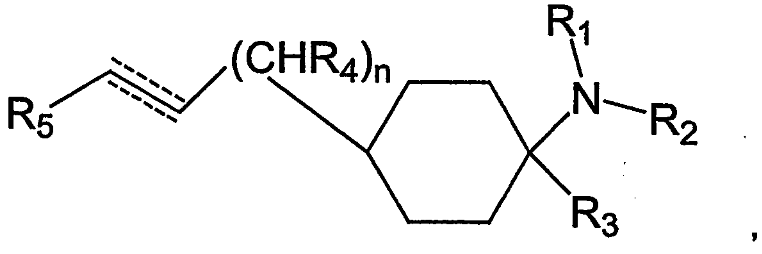

I

worin die so:

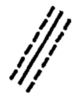

dargestellte Linie eine Einfach-, Doppel- oder Dreifach-Bindung ist,

$R^1$ und $R^2$ unabhängig voneinander für H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenen Aryl, $C_{3-8}$-Cycloalkyl oder Heteroaryl, jeweils einfach oder mehrfach substituiert oder unsubstituiert, stehen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

wobei $R^6$ H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenen Aryl, $C_{3-8}$-Cycloalkyl oder Heteroaryl, jeweils einfach oder mehrfach substituiert oder unsubstituiert, bedeutet;

$R^3$ für $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenen Aryl, $C_{3-8}$-Cycloalkyl oder Heteroaryl, jeweils unsubstituiert oder einfach oder mehrfach substituiert, steht;

$R^4$ für H, OH oder Acetyloxy, Benzyloxycarbonyloxy oder tert-Butoxycarbonyloxy ("OBoc") steht, wobei n = 0 oder 1 bedeutet $R^5$ für $C_{3-8}$-Cycloalkyl, Aryl oder Heteroaryl, jeweils unsubstituiert oder einfach oder mehrfach substituiert; $-CH_2R^{12}$, $-CH_2-CH_2R^{12}$, $-CH_2-CH_2-CH_2R^{12}$ steht

wobei $R^{12}$

$C_{3-8}$-Cycloalkyl, Aryl oder Heteroaryl, jeweils unsubstituiert oder einfach oder mehrfach substituiert, bedeutet;

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Alle diese erfindungsgemäßen Verbindungen zeigen gute Bindung an den ORL1-Rezeptor, aber auch an andere Opiatrezeptoren.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für

C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht, ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, $-CH_2-CH_2-$, $-CH_2-CH_2-CH_2-$ und $-CH_2-CH_2-CH_2-CH_2-$ zu verstehen, unter $(CH_2)_{4-5}$ ist $-CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, gebundenen Aryl- oder Heteroaryl-Rest, wobei diese Aryl und Heteroarylreste nicht selbst mit Aryl- oder Heteroaryl-Resten substituiert sein dürfen,

die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{23}$ und $R^{24}$ bedeuten zusammen $CH_2CH_2OCH_2CH_2$, $CH_2CH_2NR^{25}CH_2CH_2$ oder $(CH_2)_{3-6}$, und

der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz und das Citrat-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

Bevorzugt im Sinne dieser Erfindung sind 4-Alkyl-/4-Alkenyl-/4-Alkinylmethyl/-1-aryl-cyclohexylamin-Derivate, worin

$R^1$ und $R^2$ unabhängig voneinander für H; $C_{1-8}$-Alkyl, gesättigt oder ungesättigt, verzweigt oder unverzweigt, einfach oder mehrfach substituiert oder unsubstituiert, stehen;

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

wobei $R^6$ H; $C_{1-8}$-Alkyl, gesättigt oder ungesättigt, verzweigt oder unverzweigt, einfach oder mehrfach substituiert oder unsubstituiert, bedeutet,

vorzugsweise

$R^1$ und $R^2$ unabhängig voneinander für H; $C_{1-4}$-Alkyl, gesättigt oder ungesättigt, verzweigt oder unverzweigt, einfach oder mehrfach substituiert oder unsubstituiert, stehen; wobei $R^1$ und $R^2$ nicht beide H sein dürfen,

oder die Reste $R^1$ und $R^2$ zusammen einen Ring bilden und $(CH_2)_{4-5}$ bedeuten,

insbesondere

$R^1$ und $R^2$ unabhängig voneinander für Methyl oder Ethyl stehen oder die Reste $R^1$ und $R^2$ zusammen einen Ring bilden und $(CH_2)_5$ bedeuten.

Besonders bevorzugt sind 4-Alkyl-/4-Alkenyl-/4-Alkinylmethyl/-1-aryl-cyclohexylamin-Derivate, worin $R^1$ und $R^2$ unabhängig voneinander für $CH_3$ oder H stehen, wobei $R^1$ und $R^2$ nicht gleichzeitig H bedeuten.

Bevorzugt im Sinne dieser Erfindung sind auch 4-Alkyl-/4-Alkenyl-/4-Alkinylmethyl/-1-aryl-cyclohexylamin-Derivate, worin

$R^3$ für $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenen Aryl, $C_{3-8}$-Cycloalkyl oder Heteroaryl, jeweils unsubstituiert oder einfach oder mehrfach substituiert, steht;

vorzugsweise

$R^3$ Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, bedeutet;

insbesondere

$R^3$ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenen Phenyl, Furyl oder Thiophenyl, jeweils unsubstituiert oder einfach oder mehrfach substituiert, bedeutet.

Besonders bevorzugt sind 4-Alkyl-/4-Alkenyl-/4-Alkinylmethyl/-1-aryl-cyclohexylamin-Derivate, worin $R^3$ für Phenyl, Thiophenyl, Pyridyl oder Benzyl, jeweils substituiert oder unsubstituiert, steht, ganz besonders bevorzugt für Phenyl.

Weiterhin bevorzugt sind 4-Alkyl-/4-Alkenyl-/4-Alkinylmethyl/-1-aryl-cyclohexylamin-Derivate, worin
$R^5$ für $C_{3-8}$-Cycloalkyl, Aryl oder Heteroaryl, jeweils unsubstituiert oder einfach oder mehrfach substituiert, steht;
vorzugsweise
$R^5$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5] thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet; insbesondere
$R^5$ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

Außerdem bevorzugt sind 4-Alkyl-/4-Alkenyl-/4-Alkinylmethyl/-1-aryl-cyclohexylamin-Derivate, worin
$R^5$ für $-CH_2R^{12}$, $-CH_2-CH_2R^{12}$, $-CH_2-CH_2-CH_2R^{12}$ steht
vorzugsweise
$R^5$ $-CH_2R^{12}$, $-CH_2-CH_2R^{12}$ bedeutet
insbesondere
$R^5$ $-CH_2R^{12}$ bedeutet,
wobei
$R^{12}$ für $C_{3-8}$-Cycloalkyl, Aryl oder Heteroaryl, jeweils unsubstituiert oder einfach oder mehrfach substituiert, steht;
vorzugsweise
$R^{12}$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5] thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet;
insbesondere
$R^{12}$ für Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

Ganz besonders bevorzugt sind 4-Alkyl-/4-Alkenyl-/4-Alkinylmethyl/-1-aryl-cyclohexylamin-Derivate aus der Gruppe
Dimethyl-[1-phenyl-4-(2-p-tolylvinyl)cyclohexyl]amin Hydrochlorid, polarstes Diastereoisomer
Dimethyl-[1-phenyl-4-(2-p-tolylvinyl)cyclohexyl]amin Hydrochlorid, zweitpolarstes Diastereoisomer
Dimethyl-[1-phenyl-4-(2-p-tolylvinyl)cyclohexyl]amin Hydrochlorid, unpolarstes Diastereoisomer
{4-[2-(4-Fluorphenyl)vinyl]-1-phenylcyclohexyl}dimethylamin Hydrochlorid, unpolarstes Diastereoisomer
{4-[2-(4-Fluorphenyl)vinyl]-1-phenylcyclohexyl}dimethylamin Hydrochlorid, zweitunpolarstes Diastereoisomer
{4-[2-(4-Fluorphenyl)vinyl]-1-phenylcyclohexyl}dimethylamin Hydrochlorid, zweitpolarstes Diastereoisomer
{4-[2-(4-Fluorphenyl)vinyl]-1-phenylcyclohexyl}dimethylamin Hydrochlorid, polarstes Diastereoisomer
{4-[3-(1H-Indol-3-yl)-propenyl]-1-phenylcyclohexyl}dimethylamin Hydrochloride, polareres Diastereoisomer
{4-[3-(1H-Indol-3-yl)-propenyl]-1-phenylcyclohexyl}dimethylamin Hydrochlorid, unpolareres Diastereoisomer
3-[3-*tert*-Butoxycarbonyloxy-3-(4-dimethylamino-4-phenylcyclohexyl)-prop-1-ynyl]-indol-1-carbonsäure-tert-butylester, unpolareres Diastereoisomer
3-[3-*tert*-Butoxycarbonyloxy-3-(4-dimethylamino-4-phenylcyclohexyl)-prop-1-ynyl]-indol-1-carbonsäure-tert-butylester, polareres Diastereoisomer
3-[3-(4-Dimethylamino-4-phenyl-cyclohexyl)-3-hydroxyprop-1-ynyl]-indol-1-carbonsäure-*tert*-butylester
gegebenenfalls auch in Form ihrer Mischungen.

Die erfindungsgemäßen Substanzen wirken beispielsweise auf den im Zusammenhang mit verschiedenen Erkrankungen relevanten ORL1-Rezeptor sowie dem μ-Opiat-Rezeptor, sodass sie sich als pharmazeutischer Wirkstoff in einem Arzneimittel eignen.

Ein weiterer Gegenstand der Erfindung sind daher Arzneimittel enthaltend wenigstens ein 4-Alkyl-/4-Alkenyl-/4-Alkinylmethyl/-1-aryl-cyclohexylamin-Derivat.

Die erfindungsgemäßen Arzneimittel enthalten neben mindestens einem erfindungsgemäßen 4-Alkyl-/4-Alkenyl-/4-Alkinylmethyl/-1-aryl-cyclohexylamin-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße 4-Alkyl-/4-Alkenyl-/4-Alkinylmethyl/-1-aryl-cyclohexylamin-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen 4-Alkyl-/4-Alkenyl-/4-Alkinylmethyl/-1-aryl-cyclohexylamin-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen 4-Alkyl-/4-Alkenyl-/4-Alkinylmethyl/-1-aryl-cyclohexylamin-Derivats appliziert.

Für alle vorstehenden Formen der erfindungsgemäßen Arzneimittel ist es besonders bevorzugt, wenn das Arzneimittel neben wenigstens einem 4-Alkyl-/4-Alkenyl-/4-Alkinylmethyl/-1-aryl-cyclohexylamin-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes 4-Alkyl-/4-Alkenyl-/4-Alkinylmethyl/-1-aryl-cyclohexylamin-Derivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße 4-Alkyl-/4-Alkenyl-/4-Alkinylmethyl/-1-aryl-cyclohexylamin-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen 4-Alkyl-/4-Alkenyl-/4-Alkinylmethyl/-1-aryl-cyclohexylamin-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist daher die Verwendung eines erfindungsgemäßen 4-Alkyl-/4-Alkenyl-/4-Alkinylmethyl/-1-aryl-cyclohexylamin-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes 4-Alkyl-/4-Alkenyl-/4-Alkinylmethyl/-1-aryl-cyclohexylamin-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen 4-Alkyl-/4-Alkenyl-/4-Alkinylmethyl/-1-aryl-cyclohexylamin-Derivats, oder eines erfindungsgemäßen Arzneimittels.

Ein weiterer Gegenstand der Erfindung ist ein Verfahren zur Herstellung der erfindungsgemäßen 4-Alkyl-/4-Alkenyl-/4-Alkinylmethyl/-1-aryl-cyclohexylamin-Derivate wie in der folgenden Beschreibung und Beispielen ausgeführt.

Insbesondere geeignet ist dabei ein, im folgenden Hauptverfahren genanntes Verfahren zur Herstellung eines erfindungsgemäßen 4-Alkyl-/4-Alkenyl-/4-Alkinylmethyl/-1-aryl-cyclohexylamin-Derivats mit folgenden Schritten,
wobei $R^1$, $R^2$, $R^3$, $R^4$ und $R^5$ die für erfindungsgemäße Verbindungen gemäß Formel I angegebene Bedeutung haben, und
$R^{01}$ und $R^{02}$ unabhängig voneinander für eine Schutzgruppe stehen oder die für erfindungsgemäße Verbindungen

gemäß Formel I für $R^1$ und $R^2$ angegebene Bedeutung haben:

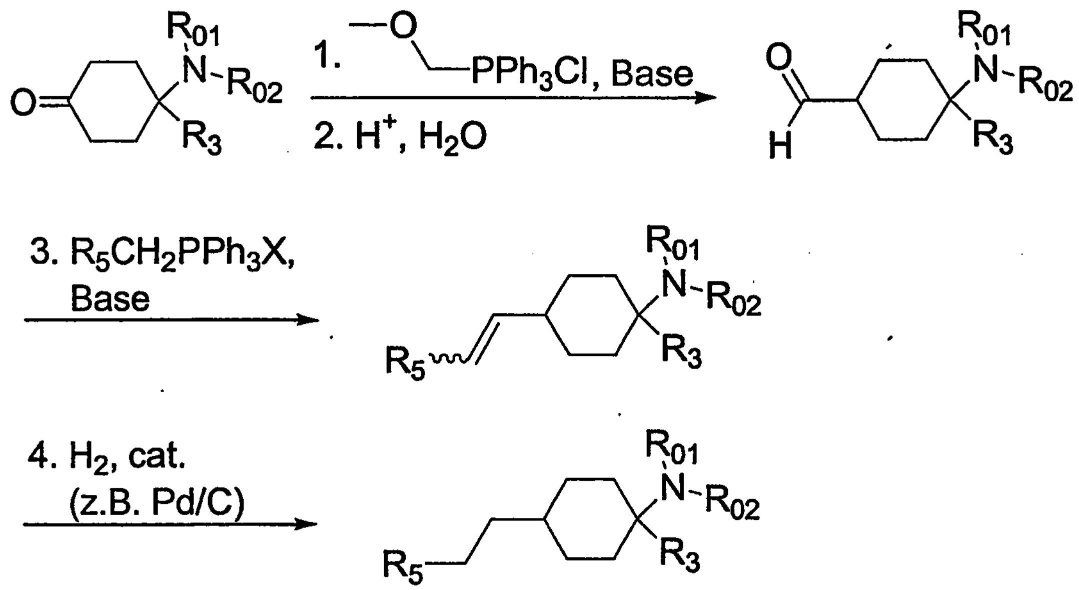


Im ersten Schritt werden 4-Aminocyclohexanon-Derivate mit Methoxytriphosphoniumchlorid und einer Base, beispielsweise Natriumhydrid, und anschließend mit wäßriger Säure, beispielsweise HCl, zu den entsprechenden Aldehyden umgesetzt.

Im zweiten Schritt werden die erhaltenen 4-Aminocyclohexancarbaldehyd-Derivate mit Wittig-Reagenzien der allgemeinen Formel $R^5CH_2PPh_3X$ in Gegenwart von Base umgesetzt und die erhaltenen Olefine gegebenenfalls zum Alkan reduziert, zum Beispiel mit $H_2$ katalytisch, beispielsweise über Pd/C.

**Alternatives allgemeines Syntheseschema:**

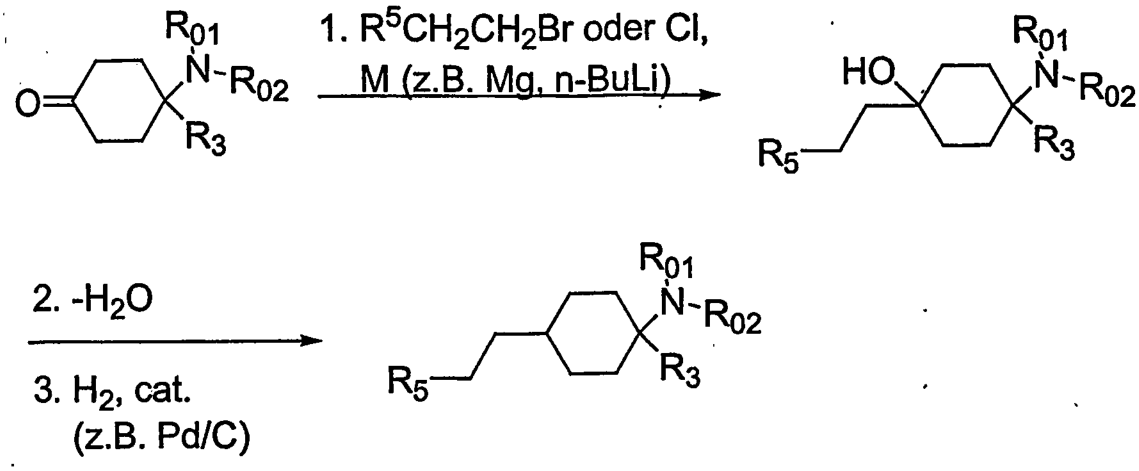


Alternativ können 4-Alkyl-/4-Alkenyl-/4-Alkinylmethyl/-1-aryl-cyclohexylamin-Derivate auch dadurch hergestellt werden, dass 4-Aminocyclohexanone mit einer Organometallverbindung, die aus $R^5CH_2CH_2Br$ oder $R^5CH_2CH_2Cl$ unter Zugabe eines geeigneten Metalls oder einer geeigneten Organometallverbindung, beispielsweise Magnesium oder n-BuLi, hergestellt werden kann, umgesetzt werden und im Anschluß durch Zugabe von Säure dehydratisiert und mit $H_2$ katalytisch, beispielsweise über Pd/C, hydriert werden.

**Allgemeines Syntheseschema (Alkin-Derivate und Alkyl-substituierte Derivate):**

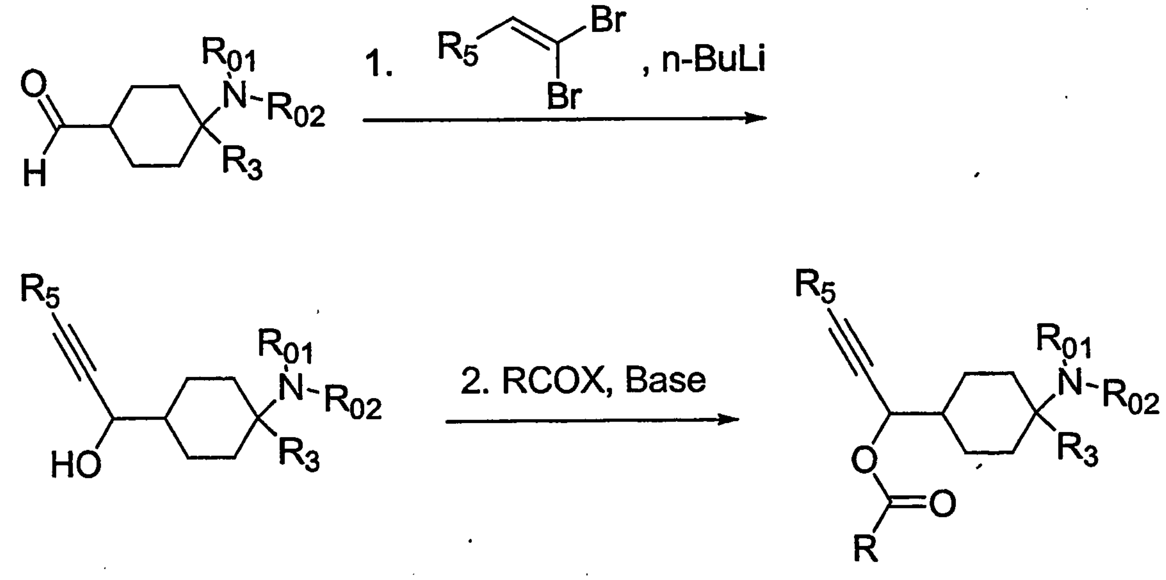


Bei der Herstellung von Alkin-Derivaten sowie an der Alkylkette substituierten Verbindungen werden 4-Aminocyclohexylcarbaldehyde mit

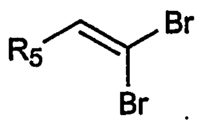

in Anwesenheit von n-BuLi zu erfindungsgemäßen 4-Alkinylmethyl-cyclohexylamin-Derivaten, bei denen $R^4$ OH bedetet. Durch Schützen der OH-Funktion mit einer Schutzgruppe sowie anschließender Hydrierung der Dreifachbindung, z. B. mit $H_2$/Pd bzw $H_2$ über Pd/C + $BaSO_4$ (Lindlar-Katalysator), kommt man zu den entsprechenden Alkyl- bzw. Alken-Verbindungen.

Die Herstellung geeigneter 4-Aminocyclohexanone ist aus der Literatur bekannt (Lednicer et al., J. Med. Chem., 23, 1980, 424-430; WO 0290317).

Die Isolierung der erfindungsgemäßen Verbindungen durch Säulenchromatographie mit Kieselgel als stationärer Phase und Ethylacetat, Methanol, aus Ethylacetat und Methanol oder Gemischen aus Ethylacetat und Diethylether als Laufmittel führt zu einer Auftrennung der unterschiedlich polaren Diastereoisomeren. Diese wurden aufgrund ihrer Laufzeit bei der Trennung als "unpolarstes Diastereoisomer" (kürzeste Laufzeit) bis "polarstes Diastereoisomer" (längste Laufzeit) charakterisiert.

Im folgenden wird die Erfindung weiter durch Beispiele erläutert, ohne sie darauf zu beschränken.

**Beispiele**

Die Ausbeuten der hergestellten Verbindungen sind nicht optimiert.

Alle Temperaturen sind unkorrigiert.

Die Angabe "Ether" bedeutet Diethylether, "EE" Ethylacetat, "DCM" Dichlormethan, "DMF" Dimethylformamid, "DMSO" Dimethylsulfoxid und "THF" Tetrahydrofuran. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

**Beispiel 1:** Dimethyl-[1-phenyl-4-(2-p-tolylvinyl)cyclohexyl]amin Hydrochlorid, polarstes Diastereoisomer

Unter Argon wurde Methoxymethyltriphenylphosphoniumchlorid (6,3 g, 18,4 mmol) in DMF (25 ml) gelöst und Natriumhydrid (60-proz. in Mineralöl, 737 mg, 18,4 mmol) zugegeben. 4-Dimethylamino-4-phenylcyclohexanon (2,0 g, 9,2 mmol), gelöst in 25 ml DMF, wurde in 30 min zugetropft und die Suspension 3 d bei RT gerührt. Zur Aufarbeitung wurde die Suspension langsam in eine mit Eiswasser gekühlte 2M HCl (50 ml) gegossen, bei RT 2 h gerührt und anschließend mit Diethylether (5 × 25 ml) und EE (6 × 20 ml) extrahiert. Die wässrige Phase wurde dann mit 1M NaOH auf pH 10-11 gebracht und mit EE (5 × 20 ml) extrahiert. Die vereinigten Extrakte wurden mit Natriumsulfat getrocknet, filtriert und eingeengt. Der Rückstand war 4-Dimethylamino-4-phenylcyclohexancarbaldehyd (2,0 g braunes Öl) im Diastereoisomerenverhältnis 55 : 45 ($^1$H-NMR).
4-Methylbenzyltriphenylphosphoniumchlorid (1,61 g, 4 mmol) wurde unter Argon in abs. DMF suspendiert, auf 0 °C abgekühlt, portionsweise Natriumhydrid (200 mg, 60-proz. in Mineralöl, 5 mmol) zugegeben und 30 min gerührt. 4-Dimethylamino-4-phenylcyclohexancarbaldehyd (463 mg, 2 mmol) wurde in DMF gelöst und innerhalb von 35 min zugetropft. Nach langsamer Erwärmung auf RT wurde 2 d gerührt. Zur Aufarbeitung wurde DMF bis auf ca. 4 ml abdestilliert, der Rückstand auf 0 °C gekühlt und unter Rühren mit 15 ml 2N HCl versetzt. Die wässrige Suspension wurde mit Ether (5 × 20 ml) extrahiert und die vereinigten Extrakte, getrocknet, filtriert und eingeengt, wobei 1,85 g Rohprodukt als klebriger beiger Feststoff erhalten wurden. Die wässrige Phase wurde mit 1M NaOH auf pH 10 gebracht und mit EE (3 × 15 ml) extrahiert. Die vereinigten Extrakte wurden getrocknet, filtriert und eingeengt, wobei weitere 113 mg Rohprodukt erhalten wurden. Die vereinigten Rohprodukte wurden durch Flash-Chromatographie an Kieselgel [170 g, Eluent: 2500 ml EE/MeOH (3:1)] gereinigt. Es wurden 120 mg des polarsten Diastereoisomers von Dimethyl-[1-phenyl-4-(2-p-tolylvinyl)cyclohexyl]amin erhalten (Smp. 130-145 °C) 110 mg (0,34 mmol) hiervon wurden in 2-Butanon/Aceton (4 ml/2 ml) in der Wärme gelöst. Bei RT wurde Chlortrimethylsilan (131 µl, 1 mmol) zugetropft und 3 h gerührt. Das Lösungsmittel wurde vollständig abdestilliert, der Rückstand mit abs. Ether überschichtet und mechanisch von der Kolbenwand gelöst. Der so erhaltene hygroskopische Feststoff wurde im Vakuum getrocknet. Das Hydrochlorid des Diastereoisomers von Dimethyl-[1-phenyl-4-(2-p-tolylvinyl)cyclohexyl]amin wurde so in einer Ausbeute von 122 mg erhalten (beiger hygroskopischer Feststoff, Smp. 140-150 °C).

**Beispiel 2:** Dimethyl-[1-phenyl-4-(2-p-tolylvinyl)cyclohexyl]amin Hydrochlorid, zweitpolarstes Diastereoisomer

Wie für Beispiel 1 beschrieben wurden auch 84 mg des zweitpolarsten Diastereoisomers erhalten (Smp. 108-120 °C). 80 mg (0,25 mmol) hiervon wurden in 2-Butanon/Methanol (5 ml/3 ml) in der Wärme gelöst, bei RT ethanolische 3,3M HCl (228 µl, 0,8 mmol) zugegeben und 60 min gerührt. Es wurde zur Trockne eingeengt, der Rückstand mit Ether überschichtet und mechanisch von der Kolbenwand gelöst. Der Feststoff wurde abgesaugt und mit Ether (2 × 2 ml) gewaschen. Das Hydrochlorid des zweitpolarsten Diastereoisomers von Dimethyl-[1-phenyl-4-(2-p-tolylvinyl)cyclohexyl]amin wurde so in einer Ausbeute von 87 mg erhalten (beiger Feststoff, Smp. 155-163 °C).

**Beispiel 3:** Dimethyl-[1-phenyl-4-(2-p-tolylvinyl)cyclohexyl]amin Hydrochlorid, unpolarstes Diastereoisomer

Wie für Beispiel 1 beschrieben wurden auch 57 mg des unpolarsten Diastereoisomers erhalten (Smp. 165-170 °C). 50 mg (0,16 mmol) hiervon wurden in Aceton/2-Butanon/Methanol (20 ml/10 ml/10 ml) in der Wärme gelöst, bei RT ethanolische 3,3M HCl (142 µl, 0,5 mmol) zugegeben und 60 min gerührt. Es wurde zur Trockne eingeengt, der Rückstand mit Ether überschichtet und mechanisch von der Kolbenwand gelöst. Der Ether wurde abdekantiert und der Rückstand im Vakuum getrocknet. Das Hydrochlorid des unpolarsten Diastereoisomers von Dimethyl-[1-phenyl-4-(2-p-tolylvinyl)cyclohexyl]amin wurde so in einer Ausbeute von 51 mg erhalten (beiger Feststoff, Smp. 208-212 °C).

**Beispiel 4:** {4-[2-(4-Fluorphenyl)vinyl]-1-phenylcyclohexyl}dimethylamin Hydrochlorid, unpolarstes Diastereoisomer

4-Fluorbenzyltriphenylphosphoniumchlorid (1,22 g, 3 mmol) wurde unter Argon in abs. THF (15 ml) suspendiert und auf -5 °C abgekühlt. Dazu wurde in THF (14 ml) gelöstes Kalium-*tert*-butanolat (337 mg, 3 mmol) getropft und 30 min bei < 0 °C nachgerührt. Anschließend wurde in THF (8 ml) gelöstes 4-Dimethylamino-4-phenylcyclohexancarbaldehyd (467 mg, 2 mmol) zugetropft und unter langsamer Erwärmung auf RT 20 h gerührt. Zur Aufarbeitung wurde eingeengt, der Rückstand mit 15 ml 1M HCl, 5 ml Wasser und 30 ml Ether versetzt. Nach 60 min Rühren befand sich zwischen der wässrigen und der organischen Phase ein weißer Feststoff, der abgesaugt und mit Ether (3 × 2 ml)

gewaschen wurde. Nach Analytik und DC war dieser Feststoff das Hydrochlorid des unpolarsten von vier möglichen Diastereoisomeren von {4-[2-(4-Fluorphenyl)vinyl]-1-phenylcyclohexyl}dimethylamin (145 mg, Smp. 255-258 °C).

**Beispiel 5:** {4-[2-(4-Fluorphenyl)vinyl]-1-phenylcyclohexyl}dimethylamin Hydrochlorid, zweitunpolarstes Diastereoisomer

Die klaren Phasen des nach Beispiel 4 erhaltenen Filtrats wurden getrennt, die wässrige mit Diethylether (5 × 20 ml) gewaschen, mit 1 N NaOH auf pH 10 eingestellt und mit EE (4 × 20 ml) extrahiert. Die vereinigten Extrakte wurden getrocknet, filtriert und eingeengt. Der Rückstand (357 mg dunkelbraunes Öl) enthielt die drei weiteren Diastereoisomeren von {4-[2-(4-Fluorphenyl)vinyl]-1-phenylcyclohexyl}dimethylamin. Die Trennung der Diastereoisomeren wurde durch Flash-Chromatographie an Kieselgel [35 g, Eluent: 350 ml EE/MeOH (5 : 1); 300 ml EE/MeOH (4 : 1); 300 ml EE/MeOH (1 : 1)] erreicht. Es wurden 66 mg des zweitunpolarsten Diastereoisomers erhalten, die in 2-Butanon (1,5 ml) gelöst, bei RT mit 3,3M ethanolischer HCl (185 µl, 0,61 mmol) versetzt und 2 h gerührt wurden. Es wurde zur Trockne eingeengt und der Rückstand in 2 Tropfen MeOH aufgenommen und mit Ether (4 ml) versetzt. Der entstandene Feststoff wurde abgesaugt und mit Ether (1 × 1 ml) gewaschen. Das Hydrochlorid des zweitunpolarsten Diastereoisomers von {4-[2-(4-Fluorphenyl)vinyl]-1-phenylcyclohexyl}dimethylamin wurde so als weißer Feststoff erhalten (60,0 mg, Smp. 159-167 °C).

**Beispiel 6:** {4-[2-(4-Fluorphenyl)vinyl]-1-phenylcyclohexyl}dimethylamin Hydrochlorid, zweitpolarstes Diastereoisomer

Wie für Beispiel 5 beschrieben wurden auch 69 mg des zweitpolarsten Diastereoisomers von {4-[2-(4-Fluorphenyl)vinyl]-1-phenylcyclohexyl}dimethylamin erhalten (gelbes Öl), die in 2-Butanon (1,5 ml) gelöst, bei RT mit 3,3M ethanolischer HCl (194 µl, 0,6 mmol) versetzt und 2 h gerührt wurden. Nach Einengen zur Trockne wurde der Rückstand in 2 Tropfen MeOH gelöst und mit Ether (8 ml) versetzt. Der entstandene Feststoff wurde abgesaugt und mit Ether (2 × 1 ml) gewaschen. Das Hydrochlorid des zweitpolarsten Diastereoisomers von {4-[2-(4-Fluorphenyl)vinyl]-1-phenylcyclohexyl}dimethylamin wurde so als weißer Feststoff erhalten (70 mg, Smp. 177-188 °C).

**Beispiel 7:** {4-[2-(4-Fluorphenyl)vinyl]-1-phenylcyclohexyl}dimethylamin Hydrochlorid, polarstes Diastereoisomer

Wie für Beispiel 5 beschrieben wurden auch 132 mg des polarsten Diastereoisomers von {4-[2-(4-Fluorphenyl)vinyl]-1-phenylcyclohexyl}dimethylamin erhalten, die in 2-Butanon/Ethanol (2 ml/10 ml) in der Wärme gelöst, bei RT mit 3,3M ethanolischer HCl (371 µl, 1,2 mmol) versetzt und 60 min gerührt wurden. Nach Einengen zur Trockne wurde der Rückstand in mit Ether (2 ml) überschichtet und mechanisch von der Kolbenwand gelöst. Der Feststoff wurde abgesaugt und mit Ether (2 × 0,5 ml) gewaschen. Das Hydrochlorid des polarsten Diastereoisomers von {4-[2-(4-Fluorphenyl)vinyl]-1-phenylcyclohexyl}dimethylamin wurde so als weißer Feststoff erhalten (126 mg, Smp. unter Zersetzung ab ca. 140 °C).

**Beispiel 8:** {4-[3-(1H-Indol-3-yl)-propenyl]-1-phenylcyclohexyl}dimethylamin Hydrochloride, polareres Diastereoisomer

[2-(1H-Indol-3-yl)ethyl]triphenylphosphoniumbromid (1,94 g, 4 mmol) wurde unter Argon in abs. THF (15 ml) suspendiert und auf -5 °C abgekühlt. In THF (15 ml) gelöstes Kalium-*tert*-butanolat (448,9 mg, 4 mmol) wurde getropft und 30 min bei < 0 °C gerührt. Dann wurde in THF (7 ml) gelöstes4-Dimethylamino-4-phenylcyclohexancarbaldehyd (463 mg, 2 mmol) innerhalb von 40 min zugetropft. Unter langsamer Erwärmung auf RT wurde 2 d gerührt. Zur Aufarbeitung wurde das THF abdestilliert und der Rückstand mit 1M HCl (20 ml), Wasser (5 ml) und Ether (30 ml) versetzt. Nach einstündigem Rühren befand sich zwischen der wässrigen und der organischen Phase eine viskose braune Schicht. Die organische Phase wurde abgetrennt, die wässrige Phase und die viskose Schicht wurden mit Dichlormethan (3 × 5 ml) kräftig ausgeschüttelt. Die klaren Phasen wurden getrennt, die vereinigten Dichlormethanextrakte wurden mit 5-proz. $NaHCO_3$-Lösung (15 ml) gewaschen, getrocknet, filtriert und eingeengt. Das erhaltene Rohprodukt wurde durch Flash-Chromatographie an Kieselgel [130 g, Eluent: 600 ml EE/MeOH (15 : 1); 700 ml EE/MeOH (10 : 1); 1500 ml EE/MeOH (5 : 1); 700 ml MeOH] gereinigt. Ein polares der 4 möglichen Diastereoisomeren wurde so in reiner Form isoliert (62 mg braunes Öl). 60 mg hiervon (0,17 mmol) wurden in 2-Butanon (1,5 ml) gelöst, mit Chlortrimethylsilan (0,33 ml, 2,6 mmol) versetzt, 30 min gerührt und zur Trockene eingeengt. Der Rückstand wurde mit Ether (2 ml) überschichtet, mechanisch von der Kolbenwand gelöst, abgesaugt und im Vakuum getrocknet. Das Hydrochlorid eines polareren Diastereoisomers von {4-[3-(1H-Indol-3-yl)-propenyl]-1-phenylcyclohexyl}-dimethylamin wurde so als hellbrauner Feststoff erhalten (65 mg, Smp. 118-122°C).

**Beispiel 9:** {4-[3-(11H-Indol-3-yl)-propenyl]-1-phenylcyclohexyl}dimethylamin Hydrochloride, unpolareres Diastereoisomer

Wie für Beispiel 8 beschrieben wurden unpolareren Diastereoisomeren von {4-[3-(1H-Indol-3-yl)-propenyl]-1-phenylcyclohexyl}dimethylamin wurden nicht sauber erhalten. Zur Reinigung wurden 750 mg unpolares Rohprodukt in 95 ml Wasser und 1 ml 85 massenprozentiger Phosphorsäure suspendiert. Die Suspension wurde mit Diethylether (3 × 20 ml) geschüttelt, Diethylether jeweils abdekantiert, dann der Feststoff abgesaugt und mit Diethylether (10 × 3 ml) gewaschen. Der Feststoff wurde getrocknet, in DMF gelöst und mit 1M NaOH (10 ml) versetzt. Die alkalische Lösung wurde mit EE (5 × 10 ml) extrahiert. Die vereinigten EE Phasen wurden getrocknet, filtriert und eingeengt. Der erhaltene Rückstand, 309 mg eines unpolareren Diastereoisomers von {4-[3-(1H-Indol-3-yl)-propenyl]-1-phenylcyclohexyl}dimethylamin (viskoses Öl) wurde in 2-Butanon (5 ml) gelöst, mit Chlortrimethylsilan (0,33 ml, 2,6 mmol) versetzt und über Nacht bei RT gerührt. Nach Zugabe von Diethylether (3 ml) und weiteren 30 min wurde das ausgefallene Hydrochlorid eines unpolareren Diastereoisomers von {4-[3-(1H-Indol-3-yl)-propenyl]-1-phenylcyclohexyl}dimethylamin abgesaugt und in einer Ausbeute von 137 mg isoliert (beigefarbener Feststoff, Smp. 138-142 °C).

**Beispiel 10:** 3-[3-tert-Butoxycarbonyloxy-3-(4-dimethylamino-4-phenylcyclohexyl)-prop-1-ynyl]indol-1-carbonsäure-tert-butylester, unpolareres Diastereoisomer UND

**Beispiel 11:** 3-[3-tert-Butoxycarbonyloxy-3-(4-dimethylamino-4-phenylcyclohexyl)-prop-1-ynyl]indol-1-carbonsäure-tert-butylester, polareres Diastereoisomer UND

**Beispiel 12:** 3-[3-(4-Dimethylamino-4-phenyl-cyclohexyl)-3-hydroxyprop-1-ynyl]-indol-1-carbonsäure-tert-butylester

Zu einer Lösung von 1H-Indol-3-carbaldehyd (10 g, 68,8 mmol) in abs. THF (150 ml) wurden bei RT unter Rühren Di-*tert*-butyldicarbonat (18,0 g, 82,6 mmol) und 4-Dimethylaminopyridin (5 mg) gegeben und 15 min bei RT gerührt. Zur Aufarbeitung wurde das Lösungsmiftelvolumen um die Hälfte reduziert, gesättigte Ammoniumchloridlösung (100 ml) und Ether (160 ml) zugegeben. Die wässrige Phase wurde mit Ether (3 × 50 ml) ausgeschüttelt und die vereinigten organischen Phasen mit Wasser (2 × 50 ml) und gesättigter Natriumchloridlösung (1 × 50 ml) gewaschen, getrocknet, filtriert und eingeengt. Es wurden 15,4 g 3-Formyl-indol-1-carbonsäure-tert-butylester als gelber Feststoff erhalten (Smp. 124-126 °C).
Zu einer Lösung von Tetrabromkohlenstoff (10,9 g, 32,7 mmol) in DCM (120 ml) wurde innerhalb von 60 min portionsweise Triphenylphosphan (17,2 g, 65,5 mmol) bei 0 °C unter Rühren gegeben und 1 h bei 0°C nachgerührt. Anschließend wurde innerhalb von 30 min in DCM (30 ml) gelöster 3-Formyl-indol-1-carbonsäure-tert-butylester (4 g, 16,4 mmol) zugetropft und 4 h bei 0 °C gerührt. Nach Erwärmen der Lösung auf RT wurde Hexan (300 ml) zugegeben und 30 min bei RT gerührt. Es wurde abgesaugt und mit Hexan nachgewaschen. Das Filtrat wurde eingeengt, wobei ein gelber Feststoff erhalten wurde. Die chromatographische Reinigung an Kieselgel erfolgte mit Cyclohexan/EE (95 : 5). Es wurden 6,26 g 3-(2,2-Dibromvinyl)-indol-1-carbonsäure-tert-butylester erhalten (weißer Feststoff, Smp. 101-104 °C). Eine Lösung von 3-(2,2-Dibromvinyl)indol-1-carbonsäure-tert-butylester (2,97 g, 7,4 mmol) in THF (20 ml) wurde bei -78 °C unter Argon vorgelegt, innerhalb von 30 min mit einer 1,6M Lösung von n-Butyllithium in Hexan (5,7 ml, 9,1 mmol) versetzt, 1 h bei -78 °C gerührt, auf Raumtemperatur gebracht und anschließend zu einer auf -78 °C gekühlten Lösung von 4-Dimethylamino-4-phenylcyclohexancarbaldehyd (580 mg, 2,5 mmol) in THF (10 ml) gegeben. Die Mischung wurde 8 h bei -78 °C gerührt, auf Raumtemperatur gebracht und mit Wasser (20 ml) versetzt. Zur Aufarbeitung wurde die wäßrige Phase mit Toluol (3 × 15 ml) extrahiert. Die vereinigten organischen Phase wurde mit gesättigter $NH_4Cl$, 0,2M NaOH und Wasser (jeweils ca. 20 ml) gewaschen, über Natriumsulfat getrocknet, filtriert und eingeengt. Eine erste chromatographische Reinigung des erhaltenen Substanzgemisches erfolgte mit Acetonitril/Methanol/1M $NH_4Cl$ (9 : 1 : 1) an Kieselgel. Nach einer zweiten säulenchromatographischen Trennung mit Methanol an Kieselgel wurden drei Verbindungen erhalten:

90 mg des unpolareren Diastereoisomers von 3-[3-tert-Butoxycarbonyloxy-3-(4-dimethylamino-4-phenylcyclohexyl)-prop-1-ynyl]indol-1-carbonsäure-tert-butylester (Smp. 111-117 °C) und

90 mg des polareren Diastereoisomers von 3-[3-tert-Butoxycarbonyloxy-3-(4-dimethylamino-4-phenylcyclohexyl)-prop-1-ynyl]indol-1-carbonsäure-tert-butylester (Smp. 75-79 °C) und

106 mg 3-[3-(4-Dimethylamino-4-phenylcyclohexyl)-3-hydroxyprop-1-inyl]indol-1-carbonsäure-tert-butylester (Smp: ab 115 °C).

**Untersuchungen zur Wirksamkeit der erfindungsgemäßen Verbindungen:**

Die in den folgenden Assays und Modellen erhobenen Daten sind in Tabelle 1 zusammengefaßt.

**Messung der ORL1-Bindung**

Die 4-Alkyl-/4-Alkenyl-/4-Alkinylmethyl/-1-aryl-cyclohexylamin-Derivate der allgemeinen Formel I wurden in einem Rezeptorbindungsassay mit $^3$H-Nociceptin/Orphaninl FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 $\mu$g Membranprotein je 200 $\mu$l Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (AmershamPharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer $K_i$-Wert in oder % Inhibition bei c=1 $\mu$M angegeben.

**Messung der $\mu$-Bindung**

Die Rezeptoraffinität zum humanen $\mu$-Opiatrezeptor wurde in einem homogenen Ansatz in Mikrotiterplatten bestimmt. Hierzu wurden Verdünnungsreihen des jeweils zu prüfenden substituierten 4-Alkyl-/4-Alkenyl-/4-Alkinylmethyl/-1-aryl-cyclohexylamin-Derivates mit einer Rezeptormembranpräparation (15-40 $\mu$g Protein pro 250 $\mu$l Inkubationsansatz) von CHO-K1-Zellen, welche den humanen p-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 $\mu$l für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 $\mu$mol/l Naloxon zugegeben. Nach Beendigung der neunzigminütigen Inkubationszeit wurden die Mikrotiterplatten für 20 Minuten bei 1000 g abzentrifugiert und die Radioaktivität in einem $\beta$-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen $\mu$-Opiatrezeptor bei einer Konzentration der Prüfsubstanzen von 1 $\mu$mol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

**Analgesieprüfung im Writhing-Test an der Maus**

Die Untersuchung auf analgetische Wirksamkeit wurde im Phenylchinon-induzierten Writhing an der Maus (modifiziert nach I.C. Hendershot und J. Forsaith (1959) J. Pharmacol. Exp. Ther. 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Äthanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen, d.h. das Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Alle Substanzen wurden in der Standarddosierung von 10 mg/kg getestet. Die prozentuale Hemmung (%Hemmung) der Writhingreaktion durch eine Substanz wurde nach folgender Formel berechnet:

$$\text{\% Hemmung} = 100 - \frac{\text{Writhingreaktionen der behandelten Tiere}}{\text{Writhingreaktionen der Kontrolltiere}} * 100$$

Für einige Substanzen wurde aus der dosisabhängigen Abnahme der Writhingreaktionen im Vergleich zu parallel untersuchten Phenylchinon-Kontrollgruppen mittels Regressionsanalyse (Auswerteprogramm Martens EDV Service, Eckental) die $ED_{50}$-Werte mit 95 % Vertrauensbereich der Writhingreaktion berechnet.

**Tabelle 1:**

| Beispiel Nr. | ORL1 Ki [nM] oder % Hemmung [1 μM] | μ Ki [nM] oder % Hemmung [1 μM] | Writhing (Maus, i.v.) $ED_{50}$ [mg/kg] oder %Hemmung (Dosis [mg/kg]) |
|---|---|---|---|
| **1** | 210 | 200 | |
| **2** | 530 | 78 | |
| **3** | 200 | 42 | 100 (10) |
| **4** | 42 | 6,9 | |
| **5** | 140 | 140 | |
| **6** | 340 | 86 | |
| **7** | 89 | 140 | |
| **8** | 55 | 79 | |
| **9** | 110 | 25 | 89 (10) |
| **10** | | 310 | |
| **11** | | 55 % | |
| **12** | 730 | 86 | |

**Parenterale Lösung eines erfindungsgemäßen 4-Alkyl-/4-Alkenyl-/4-Alkinylmethyll-1-aryl-cyclohexylamin-Derivats**

38 g eines der erfindungsgemäßen 4-Alkyl-/4-Alkenyl-/4-Alkinylmethyl/-1-aryl-cyclohexylamin-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

**1.** 4-Alkyl-/4-Alkenyl-/4-Alkinylmethyl/-1-aryl-cyclohexylamin-Derivate der allgemeinen Formel I,

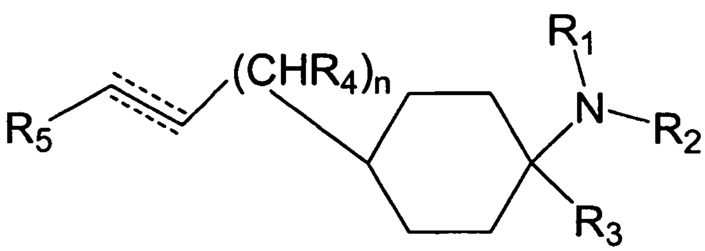
,

I

worin
die so:

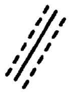

dargestellte Linie eine Einfach-, Doppel- oder Dreifach-Bindung ist,
$R^1$ und $R^2$ unabhängig voneinander für H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenen Aryl, $C_{3-8}$-Cycloalkyl oder Heteroaryl, jeweils einfach oder mehrfach substituiert oder unsubstituiert, stehen
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
wobei $R^6$ H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenen Aryl, $C_{3-8}$-Cycloalkyl oder Heteroaryl, jeweils einfach oder mehrfach substituiert oder unsubstituiert, bedeutet;
$R^3$ für $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_1$-4-Alkyl-Gruppe gebundenen Aryl, $C_{3-8}$-Cycloalkyl oder Heteroaryl, jeweils unsubstituiert oder einfach oder mehrfach substituiert, steht;
$R^4$ für H, OH, Acetyloxy, Benzyloxycarbonyloxy oder tert-Butoxycarbonyloxy ("OBoc") steht, wobei n = 0 oder 1 bedeutet
$R^5$ für $C_{3-8}$-Cycloalkyl, Aryl oder Heteroaryl, jeweils unsubstituiert oder einfach oder mehrfach substituiert; $-CH_2R^{12}$, $-CH_2-CH_2R^{12}$, $-CH_2-CH_2-CH_2R^{12}$ steht
wobei $R^{12}$ $C_{3-8}$-Cycloalkyl, Aryl oder Heteroaryl, jeweils unsubstituiert oder einfach oder mehrfach substituiert, bedeutet,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**2.** 4-Alkyl-/4-Alkenyl-/4-Alkinylmethyl/-1-aryl-cyclohexylamin-Derivate gemäß Anspruch 1, **dadurch gekennzeichnet, dass**
$R^1$ und $R^2$ unabhängig voneinander für H; $C_{1-8}$-Alkyl, gesättigt oder ungesättigt, verzweigt oder unverzweigt, einfach oder mehrfach substituiert oder unsubstituiert, stehen;
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
wobei $R^6$ H; $C_{1-8}$-Alkyl, gesättigt oder ungesättigt, verzweigt oder unverzweigt, einfach oder mehrfach substituiert oder unsubstituiert, bedeutet,
vorzugsweise
$R^1$ und $R^2$ unabhängig voneinander für H; $C_{1-4}$-Alkyl, gesättigt oder ungesättigt, verzweigt oder unverzweigt, einfach oder mehrfach substituiert oder unsubstituiert, stehen; wobei $R^1$ und $R^2$ nicht beide H sein dürfen,
oder die Reste $R^1$ und $R^2$ zusammen einen Ring bilden und $(CH_2)_{4-5}$ bedeuten,
insbesondere
$R^1$ und $R^2$ unabhängig voneinander für Methyl oder Ethyl stehen oder die Reste $R^1$ und $R^2$ zusammen einen Ring bilden und $(CH_2)_5$ bedeuten.

**3.** 4-Alkyl-/4-Alkenyl-/4-Alkinylmethyl/-1-aryl-cyclohexylamin-Derivate gemäß Anspruch 1, **dadurch gekennzeichnet, dass** $R^1$ und $R^2$ unabhängig voneinander für $CH_3$ oder H stehen, wobei $R^1$ und $R^2$ nicht gleichzeitig H bedeuten.

**4.** 4-Alkyl-/4-Alkenyl-/4-Alkinylmethyl/-1-aryl-cyclohexylamin-Derivate gemäß einem der Ansprüche 1-3, **dadurch gekennzeichnet, dass**
$R^3$ für $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenen Aryl, $C_{3-8}$-Cycloalkyl oder Heteroaryl, jeweils unsubstituiert oder einfach oder mehrfach substituiert, steht;

vorzugsweise
$R^3$ Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, bedeutet;
insbesondere
$R^3$ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenen Phenyl, Furyl oder Thiophenyl, jeweils unsubstituiert oder einfach oder mehrfach substituiert, bedeutet.

**5.** 4-Alkyl-/4-Alkenyl-/4-Alkinylmethyl/-1-aryl-cyclohexylamin-Derivate gemäß einem der Ansprüche 1 bis 3, **dadurch gekennzeichnet, dass** $R^3$ für Phenyl, Thiophenyl, Pyridyl oder Benzyl, jeweils substituiert oder unsubstituiert, steht, besonders bevorzugt für Phenyl.

**6.** 4-Alkyl-/4-Alkenyl-/4-Alkinylmethyl/-1-aryl-cyclohexylamin-Derivate gemäß einem der Ansprüche 1 bis 5, **dadurch gekennzeichnet, dass** $R^4$ für H, OH, Benzyloxycarbonyloxy, Acetyloxy oder OBoc steht.

**7.** 4-Alkyl-/4-Alkenyl-/4-Alkinylmethyl/-1-aryl-cyclohexylamin-Derivate gemäß einem der Ansprüche 1 bis 6, **dadurch gekennzeichnet, dass**
$R^5$ für $C_{3-8}$-Cycloalkyl, Aryl oder Heteroaryl, jeweils unsubstituiert oder einfach oder mehrfach substituiert, steht;
vorzugsweise
$R^5$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet;
insbesondere
$R^5$ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

**8.** 4-Alkyl-/4-Alkenyl-/4-Alkinylmethyl/-1-aryl-cyclohexylamin-Derivate gemäß einem der Ansprüche 1 bis 6, **dadurch gekennzeichnet, dass**
$R^5$ für $-CH_2R^{12}$, $-CH_2-CH_2R^{12}$, $-CH_2-CH_2-CH_2R^{12}$ steht
vorzugsweise
$R^5$ $-CH_2R^{12}$, $-CH_2-CH_2R^{12}$ bedeutet
insbesondere
$R^5$ $-CH_2R^{12}$ bedeutet, wobei
$R^{12}$ für $C_{3-8}$-Cycloalkyl, Aryl oder Heteroaryl, jeweils unsubstituiert oder einfach oder mehrfach substituiert, steht;

**9.** 4-Alkyl-/4-Alkenyl-/4-Alkinylmethyl/-1-aryl-cyclohexylamin-Derivate gemäß der Anspruch 8, **dadurch gekennzeichnet, dass**
vorzugsweise
$R^{12}$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet;
insbesondere
$R^{12}$ für Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

**10.** 4-Alkyl-/4-Alkenyl-/4-Alkinylmethyl/-1-aryl-cyclohexylamin-Derivate gemäß einem der Ansprüche 1-9 aus der Gruppe
Dimethyl-[1-phenyl-4-(2-p-tolylvinyl)cyclohexyl]amin Hydrochlorid, polarstes Diastereoisomer
Dimethyl-[1-phenyl-4-(2-p-tolylvinyl)cyclohexyl]amin Hydrochlorid, zweitpolarstes Diastereoisomer
Dimethyl-[1-phenyl-4-(2-p-tolylvinyl)cyclohexyl]amin Hydrochlorid, unpolarstes Diastereoisomer
{4-[2-(4-Fluorphenyl)vinyl]-1-phenylcyclohexyl}dimethylamin Hydrochlorid, unpolarstes Diastereoisomer
{4-[2-(4-Fluorphenyl)vinyl]-1-phenylcyclohexyl}dimethylamin Hydrochlorid, zweitunpolarstes Diastereoisomer
{4-[2-(4-Fluorphenyl)vinyl]-1-phenylcyclohexyl}dimethylamin Hydrochlorid, zweitpolarstes Diastereoisomer
{4-[2-(4-Fluorphenyl)vinyl]-1-phenylcyclohexyl}dimethylamin Hydrochlorid, polarstes Diastereoisomer
{4-[3-(1H-Indol-3-yl)-propenyl]-1-phenylcyclohexyl}dimethylamin Hydrochloride, polareres Diastereoisomer
{4-[3-(1H-Indol-3-yl)-propenyl]-1-phenylcyclohexyl}dimethylamin Hydrochloride, unpolareres Diastereoisomer
3-[3-*tert*-Butoxycarbonyloxy-3-(4-dimethylamino-4-phenylcyclohexyl)-prop-1-ynyl]-indol-1-carbonsäure-tert-butylester, unpolareres Diastereoisomer
3-[3-*tert*-Butoxycarbonyloxy-3-(4-dimethylamino-4-phenylcyclohexyl)-prop-1-ynyl]-indol-1-carbonsäure-tert-butylester, polareres Diastereoisomer
3-[3-(4-Dimethylamino-4-phenyl-cyclohexyl)-3-hydroxyprop-1-ynyl]-indol-1-carbonsäure-*tert*-butylester
gegebenenfalls auch in Form ihrer Mischungen.

**11.** Arzneimittel enthaltend mindestens ein 4-Alkyl-/4-Alkenyl-/4-Alkinylmethyl/-1-aryl-cyclohexylamin-Derivat gemäß einem der Ansprüche 1 bis 10 sowie gegebenenfalls geeignete Zusatz- und/oder Hilfsstoffe und/oder gegebenenfalls weitere Wirkstoffe.

**12.** Verwendung eines 4-Alkyl-/4-Alkenyl-/4-Alkinylmethyl/-1-aryl-cyclohexylamin-Derivats gemäß einem der Ansprüche 1 bis 10 zur Herstellung eines Arzneimittels zur Behandlung von Schmerz, insbesondere von akutem, viszeralem, neuropathischem oder chronischem Schmerz.

**13.** Verwendung eines 4-Alkyl-/4-Alkenyl-/4-Alkinylmethyl/-1-aryl-cyclohexylamin-Derivats gemäß einem der Ansprüche 1 bis 10 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

**14.** Verfahren zur Herstellung von 4-Alkyl-/4-Alkenyl-/4-Alkinylmethyl/-1-aryl-cyclohexylamin-Derivaten gemäß einem der Ansprüche 1-10, **dadurch gekennzeichnet, dass** 4-Aminocyclohexancarbaldehyd-Derivate mit Wittig-Reagenzien der allgemeinen Formel $R^5CH_2PPh_3X$ in Gegenwart von Base umgesetzt werden und die erhaltenen Olefine gegebenenfalls mit $H_2$ katalytisch, beispielsweise über Pd/C, hydriert werden.

**15.** Verfahren zur Herstellung von 4-Alkyl-/4-Alkenyl-/4-Alkinylmethyl/-1-aryl-cyclohexylamin-Derivaten gemäß einem der Ansprüche 1-10, **dadurch gekennzeichnet, dass** 4-Aminocyclohexanone mit einer Organometallverbindung, die aus $R^5CH_2CH_2Br$ oder $R^5CH_2CH_2Cl$ unter Zugabe eines geeigneten Metalls oder einer geeigneten Organometallverbindung, beispielsweise Magnesium oder n-BuLi, hergestellt werden kann, umgesetzt werden und im Anschluß durch Zugabe von Säure dehydratisiert und mit $H_2$ katalytisch, beispielsweise über Pd/C, hydriert werden.

**16.** Verfahren zur Herstellung von 4-Alkyl-/4-Alkenyl-/4-Alkinylmethyl/-1-aryl-cyclohexylamin-Derivaten gemäß einem der Ansprüche 1-10, **dadurch gekennzeichnet, dass** 4-Amino-cyclohexylcarbaldehyde mit

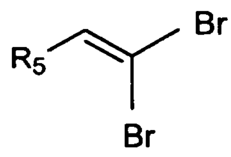

in Anwesenheit von n-BuLi zu erfindungsgemäßen 4-Alkinylmethylcyclohexylamin-Derivaten, bei denen $R^4$ OH bedetet, umgesetzt werden und gegebenenfalls die OH-Funktion mit einer Schutzgruppe versehen wird, beispielsweise Boc, sowie gegebenenfalls anschließend die Dreifachbindung hydriert wird, beispielsweise mit $H_2$/Pd oder $H_2$/Lindlar-Katalysator.

**17.** Verfahren gemäß einem der Ansprüche 14 oder 16, **dadurch gekennzeichnet, dass** die 4-Aminocyclohexancarbaldehyd-Derivate durch Umsetzung von 4-Aminocyclohexanon-Derivaten mit Methoxytriphosphoniumchlorid und einer Base, beispielsweise Natriumhydrid, und anschließend mit wäßriger Säure, beispielsweise HCl, erhalten werden.

## Claims

**1.** 4-alkyl-/4-alkenyl-/4-alkynylmethyl/-1-aryl-cyclohexylamine derivatives of general formula I

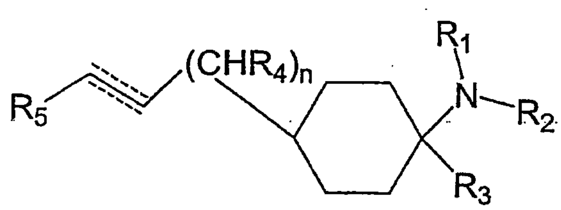

I

wherein
the line illustrated thus:

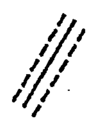

is a single, double or triple bond,
$R^1$ and $R^2$ independently of one another represent H; respectively saturated or unsaturated, branched or unbranched, singly or multiply substituted or unsubstituted $C_{1-8}$ alkyl or $C_{3-8}$ cycloalkyl; respectively singly or multiply substituted or unsubstituted aryl or heteroaryl; or respectively singly or multiply substituted or unsubstituted aryl, $C_{3-8}$ cycloalkyl or heteroaryl bound by $C_{1-3}$ alkyl
or the radicals $R^1$ and $R^2$ together form a ring and represent $CH_2CH_2OCH_2CH_2$, $CH_2CH_2NR^6CH_2CH_2$ or $(CH_2)_{3-6}$, wherein $R^6$ represents H; respectively saturated or unsaturated, branched or unbranched, singly or multiply substituted or unsubstituted $C_{1-8}$ alkyl or $C_{3-8}$ cycloalkyl; respectively singly or multiply substituted or unsubstituted aryl or heteroaryl; or respectively singly or multiply substituted or unsubstituted aryl, $C_{3-8}$ cycloalkyl or heteroaryl bound by $C_{1-3}$ alkyl;
$R^3$ represents respectively saturated or unsaturated, branched or unbranched, singly or multiply substituted or unsubstituted $C_{1-8}$ alkyl or $C_{3-8}$ cycloalkyl; respectively unsubstituted or singly or multiply substituted aryl or heteroaryl; respectively unsubstituted or singly or multiply substituted aryl, $C_{3-8}$ cycloalkyl or heteroaryl bound by a

saturated or unsaturated, branched or unbranched or substituted or unsubstituted $C_{1-4}$ alkyl group;
$R^4$ represents H, OH, acetyloxy, benzyloxycarbonyloxy or tert-butoxycarbonyloxy ("OBoc"), wherein n = 0 or 1
$R^5$ represents respectively unsubstituted or singly or multiply substituted $C_{3-8}$ cycloalkyl, aryl or heteroaryl; $-CH_2R^{12}$, $-CH_2-CH_2R^{12}$, $-CH_2-CH_2-CH_2R^{12}$
wherein $R^{12}$ represents respectively unsubstituted or singly or multiply substituted $C_{3-8}$ cycloalkyl, aryl or heteroaryl optionally in the form of their racemates, their pure stereoisomers, in particular enantiomers or diastereoisomers or in the form of mixtures of the stereoisomers, in particular the enantiomers or diastereoisomers, in any desired mixing ratio;
in the form of their acids or their bases or in the form of their salts, in particular the physiologically acceptable salts or salts of physiologically acceptable acids or cations; or in the form of their solvates, in particular the hydrates.

**2.** 4-alkyl-/4-alkenyl-/4-alkynylmethyl/-1-aryl-cyclohexylamine derivatives according to claim 1, **characterised in that** $R^1$ and $R^2$ independently of one another represent H; saturated or unsaturated, branched or unbranched, singly or multiply substituted or unsubstituted $C_{1-8}$ alkyl;
or the radicals $R^1$ and $R^2$ together form a ring and represent $CH_2CH_2OCH_2CH_2$, $CH_2CH_2NR^6CH_2CH_2$ or $(CH_2)_{3-6}$, wherein $R^6$ represents H; saturated or unsaturated, branched or unbranched, singly or multiply substituted or unsubstituted $C_{1-8}$ alkyl,
preferably
$R^1$ and $R^2$ independently of one another represent H; saturated or unsaturated, branched or unbranched, singly or multiply substituted or unsubstituted $C_{1-4}$ alkyl; wherein $R^1$ and $R^2$ must not both be H,
or the radicals $R^1$ and $R^2$ together form a ring and represent $(CH_2)_{4-5}$,
in particular
$R^1$ and $R^2$ independently of one another represent methyl or ethyl or the radicals $R^1$ and $R^2$ together form a ring and represent $(CH_2)_5$.

**3.** 4-alkyl-/4-alkenyl-/4-alkynylmethyl/-1-aryl-cyclohexylamine derivatives according to claim 1, **characterised in that** $R^1$ and $R^2$ independently of one another represent $CH_3$ or H, wherein $R^1$ and $R^2$ do not simultaneously mean H.

**4.** 4-alkyl-/4-alkenyl-/4-alkynylmethyl/-1-aryl-cyclohexylamine derivatives according to any one of claims 1 to 3, **characterised in that**
$R^3$ represents respectively unsubstituted or singly or multiply substituted $C_{3-8}$ cycloalkyl, aryl or heteroaryl; respectively unsubstituted or singly or multiply substituted aryl, $C_{3-8}$ cycloalkyl or heteroaryl bound by a saturated or unsaturated, unbranched, substituted or unsubstituted $C_{1-2}$ alkyl group;
preferably
$R^3$ represents respectively unsubstituted or singly or multiply substituted cyclopentyl, cyclohexyl, phenyl, benzyl, naphthyl, anthracenyl, thiophenyl, benzothiophenyl, furyl, benzofuranyl, benzodioxolanyl, indolyl, indanyl, benzodioxanyl, pyrrolyl, pyridyl, pyrimidyl or pyrazinyl; respectively unsubstituted or singly or multiply substituted $C_{5-6}$ cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl bound by a saturated, unbranched $C_{1-2}$ alkyl group;
in particular
$R^3$ represents respectively unsubstituted or singly or multiply substituted phenyl, furyl, thiophenyl, naphthyl, benzyl, benzofuranyl, indolyl, indanyl, benzodioxanyl, benzodioxolanyl, pyridyl, pyrimidyl, pyrazinyl or benzothiophenyl; respectively unsubstituted or singly or multiply substituted phenyl, furyl or thiophenyl bound by a saturated, unbranched $C_{1-2}$ alkyl group.

**5.** 4-alkyl-/4-alkenyl-/4-alkynylmethyl/-1-aryl-cyclohexylamine derivatives according to any one of claims 1 to 3, **characterised in that** $R^3$ represents respectively substituted or unsubstituted phenyl, thiophenyl, pyridyl or benzyl, particularly preferably phenyl.

**6.** 4-alkyl-/4-alkenyl-/4-alkynylmethyl/-1-aryl-cyclohexylamine derivatives according to any one of claims 1 to 5, **characterised in that** $R^4$ represents H, OH, benzyloxycarbonyloxy, acetyloxy or OBoc.

**7.** 4-alkyl-/4-alkenyl-/4-alkynylmethyl/-1-aryl-cyclohexylamine derivatives according to any one of claims 1 to 6, **characterised in that**
$R^5$ represents respectively unsubstituted or singly or multiply substituted $C_{3-8}$ cycloalkyl, aryl or heteroaryl;
preferably
$R^5$ represents respectively unsubstituted or singly or multiply substituted cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl,
in particular
$R^5$ represents respectively unsubstituted or singly or multiply substituted cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl.

**8.** 4-alkyl-/4-alkenyl-/4-alkynylmethyl/-1-aryl-cyclohexylamine derivatives according to any one of claims 1 to 6, **characterised in that**
$R^5$ represents $-CH_2R^{12}$, $-CH_2-CH_2R^{12}$, $-CH_2-CH_2-CH_2R^{12}$
preferably
$R^5$ represents $-CH_2R^{12}$, $-CH_2-CH_2R^{12}$,
in particular
$R^5$ represents $-CH_2R^{12}$, wherein
$R^{12}$ represents respectively unsubstituted or singly or multiply substituted $C_{3-8}$ cycloalkyl, aryl or heteroaryl.

**9.** 4-alkyl-/4-alkenyl-/4-alkynylmethyl/-1-aryl-cyclohexylamine derivatives according to claim 8, **characterised in that** preferably
$R^{12}$ represents respectively unsubstituted or singly or multiply substituted cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl,
in particular
$R^{12}$ represents respectively unsubstituted or singly or multiply substituted cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl.

**10.** 4-alkyl-/4-alkenyl-/4-alkynylmethyl/-1-aryl-cyclohexylamine derivatives according to any one of claims 1 to 9 from the group comprising
dimethyl-[1-phenyl-4-(2-p-tolylvinyl)cyclohexyl]amine hydrochloride, most polar diastereoisomer
dimethyl-[1-phenyl-4-(2-p-tolylvinyl)cyclohexyl]amine hydrochloride, second most polar diastereoisomer
dimethyl-[1-phenyl-4-(2-p-tolylvinyl)cyclohexyl]amine hydrochloride, least polar diastereoisomer
{4-[2-(4-fluorophenyl)vinyl]-1-phenylcyclohexyl}dimethylamine hydrochloride, least polar diastereoisomer
{4-[2-(4-fluorophenyl)vinyl]-1-phenylcyclohexyl}dimethylamine hydrochloride, second least polar diastereoisomer
{4-[2-(4-fluorophenyl)vinyl]-1-phenylcyclohexyl}dimethylamine hydrochloride, second most polar diastereoisomer
{4-[2-(4-fluorophenyl)vinyl]-1-phenylcyclohexyl}dimethylamine hydrochloride, most polar diastereoisomer
{4-[3-(1H-indol-3-yl)-propenyl]-1-phenylcyclohexyl}dimethylamine hydrochloride, more polar diastereoisomer
{4-[3-(1H-indol-3-yl)-propenyl]-1-phenylcyclohexyl}dimethylamine hydrochloride, less polar diastereoisomer
3-[3-tert-butoxycarbonyloxy-3-(4-dimethylamino-4-phenylcyclohexyl)-prop-1-ynyl]-indole-1-carboxylic acid-tert-butylester, less polar diastereoisomer
3-[3-tert-butoxycarbonyloxy-3-(4-dimethylamino-4-phenylcyclohexyl)-prop-1-ynyl]-indole-1-carboxylic acid-tert-butylester, more polar diastereoisomer
3-[3-(4-dimethylamino-4-phenyl-cyclohexyl)-3-hydroxyprop-1-ynyl]-indole-1-carboxylic acid-tert-butylester
optionally also in the form of their mixtures.

**11.** Pharmaceutical composition containing at least one 4-alkyl-/4-alkenyl-/4-alkynylmethyl/-1-aryl-cyclohexylamine derivatives according to any one of claims 1 to 10 and optionally suitable additives and/or auxiliaries and/or optionally further active ingredients.

**12.** Use of a 4-alkyl-/4-alkenyl-/4-alkynylmethyl/-1-aryl-cyclohexylamine derivative according to any one of claims 1 to 10 for producing a pharmaceutical composition for the treatment of pain, in particular acute, visceral, neuropathic or chronic pain.

**13.** Use of a 4-alkyl-/4-alkenyl-/4-alkynylmethyl/-1-aryl-cyclohexylamine derivative according to any one of claims 1 to

10 for producing a pharmaceutical composition for the treatment of anxiety, stress and stress-related syndromes, depression, epilepsy, Alzheimer's disease, senile dementia, general cognitive dysfunction, learning and memory disorders (as a nootropic), withdrawal symptoms, alcohol and/or drug and/or medicine abuse and/or dependency, sexual dysfunction, cardiovascular diseases, hypotension, hypertension, tinnitus, pruritus, migraine, hearing difficulties, deficient intestinal motility, impaired nutrient absorption, anorexia, obesity, locomotive disorders, diarrhoea, cachexia, urinary incontinence, or as a muscle relaxant, anti-convulsive or anaesthetic or for co-administration in treatment with an opioid analgesic or anaesthetic, for diuresis or anti-natriuresis, anxiolysis, for modulation of motor activity, for modulation of neurotransmitter release and treatment of neurodegenerative diseases associated therewith, for the treatment of withdrawal symptoms and/or for reducing opioid addiction potential.

**14.** Method for producing 4-alkyl-/4-alkenyl-/4-alkynylmethyl/-1-aryl-cyclohexylamine derivatives according to any one of claims 1 to 10, **characterised in that** 4-aminocyclohexane carbaldehyde derivatives are reacted with Wittig reagents of general formula $R^5CH_2PPh_3X$ in the presence of base and the olefins obtained are hydrated, optionally catalytically with $H_2$, for example using Pd/C.

**15.** Method for producing 4-alkyl-/4-alkenyl-/4-alkynylmethyl/-1-aryl-cyclohexylamine derivatives according to any one of claims 1 to 10, **characterised in that** 4-aminocyclohexanones are reacted with an organometallic compound, which may be produced from $R^5CH_2CH_2Br$ or $R^5CH_2CH_2Cl$ with the addition of a suitable metal or a suitable organometallic compound, for example magnesium or n-BuLi, and are subsequently dehydrated by addition of acid and catalytically hydrated with $H_2$, for example using Pd/C.

**16.** Method for producing 4-alkyl-/4-alkenyl-/4-alkynylmethyl/-1-aryl-cyclohexylamine derivatives according to any one of claims 1 to 10, **characterised in that** 4-aminocyclohexyl carbaldehydes are reacted with

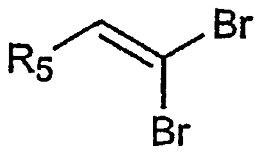

in the presence of n-BuLi to form 4-alkynylmethyl-cyclohexylamine derivatives according to the invention, wherein $R^4$ represents OH and optionally the OH function is provided with a protecting group, for example Boc, and the triple bond is subsequently optionally hydrated, for example using $H_2$/Pd or $H_2$/Lindlar catalyst.

**17.** Method according to either claim 14 or claim 16, **characterised in that** the 4-aminocyclohexane carbaldehyde derivatives are obtained by reacting 4-aminocyclohexanone derivatives with methoxytriphosphonium chloride and a base, for example sodium hydride, and subsequently with aqueous acid, for example HCl.

## Revendications

**1.** Dérivés de 4-alkyl-/4-alcényl-/4-alcynylméthyl/-1-aryl-cyclohexylamines de formule générale I

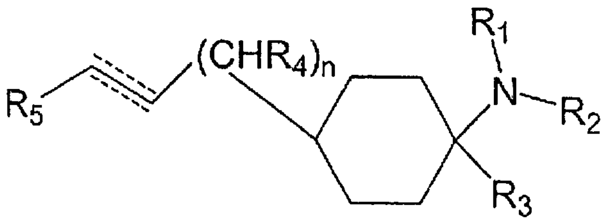

I

dans laquelle la ligne représentée par :

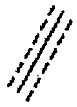

désigne une liaison simple, double ou triple,

$R^1$ et $R^2$ représentent, indépendamment l'un de l'autre, H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un radical alkyle en $C_1$ à $C_3$ et chacun substitué une ou plusieurs fois ou non substitué,

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ représentant H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un radical alkyle en $C_1$ à $C_3$, chacun substitué une ou plusieurs fois ou non substitué ;

$R^3$ désigne un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste aryle, cycloalkyle en $C_3$ à $C_8$ ou hétéroaryle lié par l'intermédiaire d'un groupe alkyle en $C_1$ à $C_4$ saturé ou non saturé, ramifié ou non ramifié, substitué ou non substitué, chacun étant non substitué ou substitué une ou plusieurs fois ;

$R^4$ représente H, OH ou un reste acétyloxy, benzyloxycarbonyloxy ou tertiobutoxycarbonyloxy (« OBoc »), n ayant la valeur 0 ou 1,

$R^5$ est un reste cycloalkyle en $C_3$ à $C_8$, aryle ou hétéroaryle, chacun non substitué ou substitué une ou plusieurs fois ; un reste $-CH_2R^{12}$, $CH_2-CH_2R^{12}$, $-CH_2-CH_2-CH_2R^{12}$,

où $R^{12}$ représente un reste cycloalkyle en $C_3$ à $C_8$, aryle ou hétéroaryle, chacun non substitué ou substitué une ou plusieurs fois ;

le cas échéant sous forme de leurs racémates, de leurs stéréoisomères purs, en particulier des énantiomères ou des diatéréo-isomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables ou de sels d'acides ou de cations physiologiquement acceptables ; ou sous forme de leurs produits de solvatation, en particulier de leurs hydrates.

**2.** Dérivés de 4-alkyl-/4-alcényl-/4-alcynylméthyl/-1-aryl-cyclohexylamines suivant la revendication 1, **caractérisés en ce que**

$R^1$ et $R^2$ représentent, indépendamment l'un de l'autre, H ; un reste alkyle en $C_1$ à $C_8$, saturé ou non saturé, ramifié ou non ramifié, substitué une ou plusieurs fois ou non substitué ;

ou bien les restes $R^1$ et $R^2$ forment ensemble un noyau et désignent un groupe $CH_2CH_2OCH_2CH_2$, $CH_2CH_2NR^6CH_2CH_2$ ou $(CH_2)_{3-6}$,

où $R^6$ représente H ; un reste alkyle en $C_1$ à $C_8$, saturé ou non saturé, ramifié ou non ramifié, substitué une ou plusieurs fois ou non substitué,

avantageusement

$R^1$ et $R^2$ représentent, indépendamment l'un de l'autre, H; un reste alkyle en $C_1$ à $C_4$, saturé ou non saturé, ramifié ou non ramifié, substitué une ou plusieurs fois ou non substitué ; $R^1$ et $R^2$ ne pouvant pas tous deux représenter H, ou bien les restes $R^1$ et $R^2$ forment ensemble un noyau et représentent $(CH_2)_{4-5}$,

en particulier

$R^1$ et $R^2$ représentent indépendamment l'un de l'autre un reste méthyle ou éthyle ou bien les restes $R^1$ et $R^2$ forment ensemble un noyau et représentent $(CH_2)_5$.

**3.** Dérivés de 4-alkyl-/4-alcényl-/4-alcynylméthyl/-1-aryl-cyclohexylamines suivant la revendication 1, **caractérisés en ce que** $R^1$ et $R^2$ représentent, indépendamment l'un de l'autre, $CH_3$ ou H, $R^1$ et $R^2$ ne représentant pas en même temps H.

**4.** Dérivés de 4-alkyl-/4-alcényl-/4-alcynylméthyl-/1-aryl-cyclohexylamines suivant l'une des revendications 1 à 3, **caractérisés en ce que**

$R^3$ est un reste cycloalkyle en $C_3$ à $C_8$, aryle ou hétéroaryle, chacun non substitué ou substitué une ou plusieurs

fois; un reste aryle, cycloalkyle en $C_3$ à $C_8$ ou hétéroaryle lié par l'intermédiaire d'un groupe alkyle en $C_1$ ou $C_2$ saturé ou non saturé, non ramifié, substitué ou non substitué, chacun étant non substitué ou substitué une ou plusieurs fois ;
avantageusement
$R^3$ représente un reste cyclopentyle, cyclohexyle, phényle, benzyle, naphtyle, anthracényle, thiophényle, benzothiophényle, furyle, benzofurannyle, benzodioxolannyle, indolyle, indanyle, benzodioxannyle, pyrrolyle, pyridyle, pyrimidyle ou pyrazinyle, chacun non substitué ou substitué une ou plusieurs fois ; un reste, lié par l'intermédiaire d'un groupe alkyle en $C_1$ ou $C_2$ saturé, non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ;
en particulier
$R^3$ désigne un reste phényle, furyle, thiophényle, naphtyle, benzyle, benzofurannyle, indolyle, indanyle, benzodioxannyle, benzodioxolannyle, pyridyle, pyrimidyle, pyrazinyle ou un reste benzothiophényle, chacun non substitué ou substitué une ou plusieurs fois ; un reste phényle, furyle ou thiophényle lié par l'intermédiaire d'un groupe alkyle en $C_1$ ou $C_2$ saturé, non ramifié, chacun étant non substitué ou substitué une ou plusieurs fois.

5. Dérivés de 4-alkyl-/4-alcényl-/4-alcynylméthyl/-1-aryl-cyclohexylamines suivant l'une des revendications 1 à 3, **caractérisés en ce que** $R^3$ représente un reste phényle, thiophényle, pyridyle ou benzyle, chacun substitué ou non substitué, notamment un reste phényle.

6. Dérivés de 4-alkyl-/4-alcényl-/4-alcynylméthyl/-1-aryl-cyclohexylamines suivant l'une des revendications 1 à 5, **caractérisés en ce que** $R^4$ représente H, OH, un reste benzyloxycarbonyloxy, acétyloxy ou OBoc.

7. Dérivés de 4-alkyl-/4-alcényl-/4-alcynylméthyl/-1-aryl-cyclohexylamines, suivant l'une des revendications 1 à 6, **caractérisés en ce que**
$R^5$ représente un reste cycloalkyle en $C_3$ à $C_8$, aryle ou hétéroaryle, chacun non substitué ou substitué une ou plusieurs fois ;
avantageusement
$R^5$ représente un reste cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle, ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;
en particulier
$R^5$ désigne un reste cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

8. Dérivés de 4-alkyl-/4-alcényl-/4-alcynylméthyl/-1-aryl-cyclohexylamines, suivant l'une des revendications 1 à 6, **caractérisés en ce que**
$R^5$ est un groupe $-CH_2R^{12}$, $-CH_2-CH_2R^{12}$, $-CH_2CH_2-CH_2R^{12}$,
avantageusement,
$R^5$ est un groupe $-CH_2R^{12}$, $-CH_2-CH_2R^{12}$,
en particulier,
$R^5$ est un groupe $-CH_2R^{12}$,
$R^{12}$ étant un reste cycloalkyle en $C_3$ à $C_8$, aryle ou hétéroaryle, chacun non substitué ou substitué une ou plusieurs fois.

9. Dérivés de 4-alkyl-/4-alcényl-/4-alcynylméthyl/-1-aryl-cyclohexylamines suivant la revendication 8, **caractérisés en ce que**
avantageusement
$R^{12}$ est un reste cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle,

phtalazinyle ou quinazolinyle, chacun non substitué ou substitué une ou plusieurs fois ;
en particulier
$R^{12}$ est un reste cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**10.** Dérivés de 4-alkyl-/4-alcényl-/4-alcynylméthyl-/1-aryl-cyclohexylamines suivant l'une des revendications 1 à 9, du groupe
Chlorhydrate de diméthyl-[1-phényl-4-(2-p-tolylvinyl)-cyclohexyl]-amine, diastéréoisomère le plus polaire
Chlorhydrate de diméthyl-[1-phényl-4-(2-p-tolylvinyl)-cyclohexyl]-amine, second diastéréoisomère le plus polaire
Chlorhydrate de diméthyl-[1-phényl-4-(2-p-tolylvinyl)-cyclohexyl]-amine, diastéréoisomère le moins polaire
Chlorhydrate de {4-[2-(4-fluorophényl)-vinyl]-1-phénylcyclohexyl}-diméthylamine, diastéréoisomère le moins polaire
Chlorhydrate de {4-[2-(4-fluorophényl)-vinyl]-1-phénylcyclohexyl}-diméthylamine, second diastéréoisomère le moins polaire
Chlorhydrate de {4-[2-(4-fluorophényl)-vinyl]-1-phénylcyclohexyl}-diméthylamine, second diastéréoisomère le plus polaire
Chlorhydrate de {4-[2-(4-fluorophényl)-vinyl]-1-phénylcyclohexyl}-diméthylamine, diastéréoisomère le plus polaire
Chlorhydrate de {4-[3-(1H-indole-3-yl)-propényl]-1-phénylcyclohexyl}-diméthylamine, diastéréoisomère le plus polaire
Chlorhydrate de {4-[3-(1H-indole-3-yl)-propényl]-1-phénylcyclohexyl}-diméthylamine, diastéréoisomère le moins polaire
Ester tertiobutylique d'acide 3-[3-tertiobutoxycarbonyloxy-3-(4-diméthylamino-4-phénylcyclohexyl)-prop-1-ynyl]-indole-1-carboxylique, diastéréoisomère le moins polaire
Ester tertiobutylique d'acide 3-[3-tertiobutoxycarbonyloxy-3-(4-diméthylamino-4-phénylcyclohexyl)-prop-1-ynyl]-indole-1-carboxylique, diastéréoisomère le plus polaire
Ester tertiobutylique d'acide 3-[3-(4-diméthylamino-4-phényl-cyclohexyl)-3-hydroxyprop-1-ynyl]-indole-1-carboxylique,
le cas échéant également sous forme de leurs mélanges.

**11.** Médicament contenant au moins un dérivé de 4-alkyl-/4-alcényl-/4-alcynylméthyl/-1-aryl-cyclohexylamine suivant l'une des revendications 1 à 10, ainsi que, le cas échéant, des additifs et/ou des substances auxiliaires convenables et/ou le cas échéant d'autres substances actives.

**12.** Utilisation d'un dérivé de 4-alkyl-/4-alcényl-/4-alcynylméthyl/-1-aryl-cyclohexylamine suivant l'une des revendications 1 à 10, pour la préparation d'un médicament destiné au traitement de la douleur, en particulier d'une douleur aiguë, viscérale, neuropathique ou chronique.

**13.** Utilisation d'un dérivé de 4-alkyl-/4-alcényl-/4-alcynylméthyl/-1-aryl-cyclohexylamine suivant l'une des revendications 1 à 10, pour la préparation d'un médicament destiné au traitement d'états d'angoisse, de stress et de syndromes liés au stress, de dépressions, de l'épilepsie, de la maladie d'Alzheimer, de la démence sénile, de dysfonctions cognitives générales, de troubles de l'apprentissage et de la mémoire (comme nootrope), de phénomènes de privation, d'abus de l'alcool et/ou des drogues et/ou de médicaments et/ou de dépendance à leur égard, de dysfonctionnements sexuels, de maladies cardiovasculaires, d'hypotension, d'hypertension, d'acouphènes, du prurit, de la migraine, de troubles de l'audition, de la motilité intestinale insuffisante, de troubles de l'absorption de nourriture, de l'anorexie, de l'obésité, de troubles de la locomotion, de diarrhée, de la cachexie, d'incontinence d'urine, ou comme relaxant musculaire, anticonvulsif ou anesthésique ou en vue d'une co-administration en cas de traitement avec un analgésique opioïde ou un anesthésique, pour la diurèse ou l'antinatriurèse, l'anxiolyse, pour la modulation de l'activité motrice, pour la modulation de la sécrétion de neurotransmetteurs et pour le traitement de maladies neurodégénératives qui y sont liées, pour le traitement de phénomènes de privation et/ou pour la réduction du risque de pharmacodépendance à l'égard des opioïdes.

**14.** Procédé de production de dérivés de 4-alkyl-/4-alcényl-/4-alcynylméthyl/-1-aryl-cyclohexylamines suivant l'une des revendications 1 à 10, **caractérisé en ce que** des dérivés de 4-aminocyclohexanecarbaldéhyde sont amenés à réagir avec des réactifs de Wittig de formule générale $R^5CH_2PPh_3X$ en présence d'une base et les oléfines obtenues sont hydrogénées par voie catalytique éventuellement avec $H_2$, par exemple sur Pd/C.

**15.** Procédé de production de dérivés de 4-alkyl-/4-alcényl-/4-alcynylméthyl/-1-aryl-cyclohexylamines suivant l'une des revendications 1 à 10, **caractérisé en ce que** des 4-aminocyclohexanones sont amenés à réagir avec un composé

organométallique qui peut être préparé à partir de $R^5CH_2CH_2Br$ ou de $R^5CH_2CH_2Cl$ avec addition d'un métal convenable ou d'un composé organométallique convenable, par exemple le magnésium ou n-BuLi, puis le produit de réaction est déshydraté par addition d'acide et hydrogéné par voie catalytique avec $H_2$, par exemple sur Pd/C.

**16.** Procédé de production de dérivés de 4-alkyl-/4-alcényl-/4-alcynylméthyl/-1-aryl-cyclohexylamines suivant l'une des revendications 1 à 10, **caractérisé en ce que** des dérivés de 4-aminocyclohexanecarbaldéhyde sont amenés à réagir avec

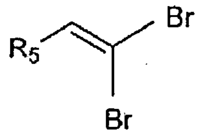

en présence de n-BuLi pour produire des dérivés de 4-alcynylméthylcyclohexylamines selon l'invention, dans lesquels $R^4$ désigne OH, et le cas échéant, la fonction OH est pourvue d'un groupe protecteur, par exemple Boc, puis le cas échéant la triple liaison est hydrogénée, par exemple avec $H_2$/Pd ou $H_2$/catalyseur de Lindlar.

**17.** Procédé suivant l'une des revendications 14 ou 16, **caractérisé en ce que** les dérivés de 4-aminocyclohexanecarbaldéhyde sont obtenus par réaction de dérivés de 4-aminocyclohexanone avec le chlorure de méthoxytriphosphonium et une base, par exemple l'hydrure de sodium, puis avec un acide aqueux, par exemple HCl.